# DEMANDE DE BREVET EUROPEEN

(11) **EP 1 459 732 A1**
(43) Date de publication de la demande: **22.09.2004**
(21) Numéro de dépôt: 04290634.7
(22) Date de dépôt: 09.03.2004
(51) Int. Cl.: A61K 7/13

(54) **Composition tinctoriale comprenant au moins une base d'oxydation hétérocyclique et au moins un coupleur 2,3,5-triaminopyridine**

(30) Priorité: 13.03.2003 FR 0303115
(71) Demandeur: L'OREAL, 75008 Paris (FR)
(72) Inventeur: Kravtchenko, Sylvain, 92600 Asnieres (FR); Lagrange, Alain, 77700 Coupvray (FR); Vidal, Laurent, 75013 Paris (FR); Fadli, Aziz, 77500 Chelles (FR)
(74) Mandataire: Fevrier, Murielle

(57) **Abrégé**

L'invention a pour objet une composition tinctoriale comprenant au moins une base d'oxydation hétérocyclique et au moins un coupleur du type 2,3,5-triaminopyridine.

L'invention a aussi pour objet l'utilisation de cette composition pour la teinture des fibres kératiniques ainsi que le procédé de teinture mettant en oeuvre cette composition.

La composition de la présente invention permet en particulier d'obtenir des nuances foncées telles que des nuances grises à noires.

## Description

L'invention a pour objet une composition tinctoriale comprenant au moins une base d'oxydation hétérocyclique et au moins un coupleur 2,3,5-triaminopyridine. L'invention a aussi pour objet l'utilisation de cette composition pour la teinture des fibres kératiniques ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des compositions tinctoriales comprenant une base d'oxydation hétéroaromatique pour la teinture de fibres kératiniques. Par exemple, le brevet US 5 380 340 décrit des bases d'oxydation du type pyrazolopyrimidine. La demande de brevet FR 2 750 048 décrit des compositions pour la teinture d'oxydation des fibres kératiniques comprenant une base d'oxydation du type pyrazolo-[1,5-a]-pyrimidine. Ces bases d'oxydation permettent d'obtenir des nuances variées, en particulier dans le domaine des rouges. Cependant, elles ne permettent pas d'obtenir des nuances foncées, en particulier des nuances grises à noires.

Par ailleurs, il est connu d'utiliser dans le domaine de la coloration des fibres kératiniques des coupleurs pyridiniques. Par exemple, le document US 4784667 décrit des coupleurs 6-alcoxy 2,3-diaminopyridine. Le document FR 2 779 952 décrit des compositions contenant des bases pyrazolo-pyrimidines et des coupleurs pyridiniques.

Le but de la présente invention est de développer de nouvelles compositions tinctoriales permettant d'obtenir une coloration des fibres kératiniques dans des nuances très variées sombres ou chromatiques, en particulier des nuances variant du gris au noir, la coloration étant peu sélective présentant une bonne ténacité vis à vis des différentes agressions extérieures que peuvent subir les cheveux.

Ce but est atteint avec la présente invention qui a pour objet une composition tinctoriale comprenant, dans un milieu de teinture approprié, au moins une base hétérocyclique et au moins un coupleur pyridinique substitué en position 2,3,5 par un radical amino.

L'invention a aussi pour objet un procédé de teinture des fibres kératiniques à partir de cette composition.

Un autre objet de l'invention est l'utilisation de la composition de la présente invention pour la teinture des fibres kératiniques, en particulier les fibres kératiniques humaines telles que les cheveux.

La composition de la présente invention permet en particulier d'obtenir une coloration de fibres kératiniques dans des nuances foncées telles que des nuances grises à noires qui présentent notamment l'avantage de ne pas virer après lavage ou exposition à la lumière.

Dans le cadre de la présente invention, on entend par alkyle, des radicaux linéaires ou ramifiés, par exemple méthyle, éthyle, n-propyle, iso-propyle, butyle, etc.

Un radical aryle en C₆-C₁₈ est un radical par exemple choisi parmi un radical benzénique, un radical naphtalénique.

Selon un mode de réalisation particulier, le coupleur pyridinique est un coupleur 2,3,5-triaminopyridinique de formule (1) : 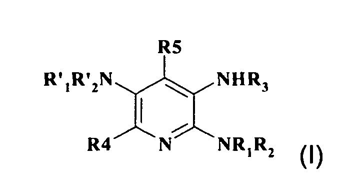 dans laquelle
- R₁, R₂, R'₁, R'₂, R₃ et R₅ représentent, indépendamment un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono di ou tri alkyle(C1-C4)amino, aryle, alcoxyC₁-C₃ ou hétérocycle ; un radical aryl C₆-C₁₈, éventuellement substitué par un ou plusieurs radicaux amino, hydroxy, alcoxy(C₁-C₃) ou alkyl(C₁-C₆) ; un radical alcényle en C₂-C₆ ; un radical carboxyalkyl(C₁-C₆) ou un radical alkylthio(C₁-C₆), ou
- R₁ et R₂, et/ou R'₁, R'₂ ensemble, forment avec l'atome d'azote auquel ils sont rattachés un hétérocycle ayant de 3 à 18 chaînons, pouvant contenir un ou plusieurs hétéroatomes supplémentaires choisis parmi les atomes d'oxygène, d'azote ou de soufre, cet hétérocycle pouvant être substitué par un ou plusieurs radicaux alkyles en C₁-C₆, amino ou hydroxyalkyle(C₁-C₄), le cycle ne comportant pas de liaison peroxyde, ni de radicaux diazo ou nitroso ;
- R₄ représente un atome d'hydrogène, un radical hydroxy, alcoxy, aryloxy, un atome d'halogène, un radical -OSCO₂R", un radical -OCOR", un radical trifluoroalcoxy en C₁-C₃, avec R" représentant un atome d'hydrogène ou un radical alkyle.

Plus particulièrement, le coupleur pyridinique correspond à la formule (I) ainsi que les sels d'addition correspondants : 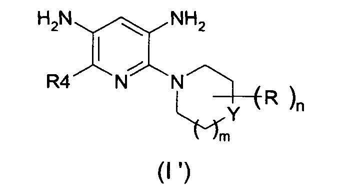 dans laquelle:
- R représente :
   - un atome d'halogène (tel que fluor, chlore, brome) ;
   - un radical alkyle en C₁-C₄ éventuellement substitué par un ou plusieurs radicaux choisis parmi les radicaux hydroxy, carboxy, alcoxycarbonyle en C₁-C₄, carboxamido, alkyl(C₁-C₄)sulfonyle, un radical alcoxy en C₁-C₄, un radical alkyl(C₁-C₄)sulfonamido, un radical NR₆R₇ ;
   - un radical carboxy ;
   - un radical alcoxycarbonyle en C₁-C₄ ;
   - un radical carboxamido ; (NH₂CO-)
   - un radical alkyl en C₁-C₄ carboxamido ; (alkylHNCO- ou (alkyl)₂NHCO-)
   - un radical sulfinique (HSO₂-)
   - un radical alkyl(C₁-C₄)sulfonyle ; (alkyl-SO₂-)
   - un radical alkyl(C₁-C₄)sulfonamido ; (alkyl-SO₂NH-)
   - un radical hydroxy ;
   - un radical alcoxy en C₁-C₄ ;
   - un radical hydroxyalcoxy en C₂-C₄ ;
   - un radical amino ou mono- ou di- amino alcoxy;
   - un radical thioéther en C₁-C₄ ;
   - un radical alkyl(C₁-C₄)sulfoxyde ; (alkyl-SO-) ;
   - un radical sulfonique (-SO3H) ;
   - un radical NR₈R₉ ;
- R₆, R₇, R₈ et R₉ représentent, identiques ou différents, un atome d'hydrogène ; un radical alkyl(C₁-C₄)sulfonyle ; un radical alkyle(C₁-C₄)carbonyle dans lequel le radical alkyle peut être substitué par un ou plusieurs hydroxy ; un radical arylcarbonyle, le radical aryle pouvant être substitué par un radical choisi parmi hydroxy, alcoxy en C₁-C₄, amino ou (di)alkyl(C₁-C₄)amino ; un radical carboxamido ; un radical alkyle en C₁-C₄ éventuellement substitué par un ou plusieurs radicaux choisis parmi les radicaux hydroxy, alcoxy en C₁-C₄, alkyl(C₁-C₄)sulfonyle, alkyl(C₁-C₄)sulfonamido, carboxy, carboxamido, alkyl(C₁-C₄)sulfoxyde, amino ,(di)(C₁-C₄)alkylamino ou (poly)hydroxyalkylamino en C₂-C₄ ;
- R₄ représente un atome d'hydrogène; un radical alcoxy en C₁-C₄ éventuellement substitué par un ou plusieurs radicaux hydroxy, alcoxy en C₁-C₂,
- n est un nombre entier compris entre 0 et 7, inclus
- m est 0, 1 ou 2,
- Y représente un atome d'oxygène, un radical CR₁₁ ou un radical NR₁₀ où R₁₀ a la même signification que R₆ et R₁₁, identiques ou différents, représentent l'hydrogène ou ont la même signification que R.

A titre d'exemple, R représente un radical alcoxy en C1-C4 éventuellement substitué par un ou plusieurs radicaux hydroxy, alcoxy en C1-C2, amino ou (di)alkylamino ; un radical hydroxy ; un radical amino ; un radical (di)alkylamino ; un radical alkyle en C1-C2 éventuellement substitué par un hydroxy, amino.

A titre d'exemple, R₄ représente un atome d'hydrogène ou un radical alcoxy.

De préférence, au moins un des radicaux R₁₁ représente un atome d'hydrogène.

Dans la formule (1'), n représente de préférence 0 ou 1.

R₆, R₇, R₈, R₉ et R₁₀ peuvent représenter, identiques ou différents, un atome d'hydrogène, un radical carboxamido, un radical alkyle en C₁-C₄ éventuellement substitué par un ou plusieurs radicaux choisis parmi les radicaux hydroxy, alcoxy en C₁-C₄, carboxamido, amino, (di)(C₁-C₄)alkylamino, (poly)hydroxyalkylamino en C₂-C₄.

A titre d'exemple, R₆, R₇, R₈, R₉ et R₁₀ représentent, identiques ou différents, un atome d'hydrogène, un radical méthyle, un éthyle, 2-carboxyéthyle, 2-hydroxyéthyle, 3-hydroxypropyle, 2,3-dihydroxypropyle, 2-hydroxy-3-aminopropyle, 3-hydroxy-2-aminopropyle. De préférence, R₆, R₇, R₈, R₉ et R₁₀ représentent, identiques ou différents un atome d'hydrogène, un radical méthyle, un radical 2-hydroxyéthyle, un radical 2,3-dihydroxypropyle.

Dans la formule (I'), l'azote en position 2 du cycle, Y et m forment de préférence un radical hétérocyclique choisi parmi les pyrrolidines, les pipéridines, les homopipéridines, les pipérazines, les homopipérazines, les diazépanes (ou 1,4-diazacycloheptane),

A titre d'exemple, l'hétérocycle peut être choisi parmi la pyrrolidine, la 2,5-diméthylpyrrolidine, la 2-méthylpyrrolidine la proline, la 3-hydroxyproline, la 4-hydroxyproline, la 2,4-dicarboxypyrrolidine, la 2-hydroxyméthylpyrrolidine, la 3-hydroxy-2-hydroxyméthylpyrrolidine, la 2,5-di(hydroxyméthyl)pyrrolidine, la 2-carboxamidopyrrolidine, la 3-hydroxy-2-carboxamidopyrrolidine, la 2-(diméthylcarboxamido)pyrrolidine, la 2-(diméthylcarboxamido)-3-hydroxy-pyrrolidine, la 3,4-dihydroxy-2-hydroxyméthyl pyrrolidine, la 3-hydroxypyrrolidine, la 3,4-dihydroxypyrrolidine, la 3-aminopyrrolidine, la 3-méthylaminopyrrolidine, la 3-diméthylaminopyrrolidine, la 4-amino-3-hydroxy pyrrolidine, la 4-méthylamino-3-hydroxypyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-(diméthylcarboxamido)pipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, la pipérazine, la 4-méthylpipérazine le diazépane, N-méthyl homopipérazine, la N β-hydroxyéthylhomopipérazine et leurs sels d'addition. De préférence, l'hétérocycle est choisi parmi la pyrrolidine, la 2-méthylpyrrolidine, la 3-hydroxypyrrolidine, la 3-aminopyrrolidine, la 3-(méthylsulfonylamino)-pyrrolidine, la proline, la 3-hydroxyproline, la pipéridine, l'hydroxypipéridine, l'homopipéridine, la 4-méthylpipérazine, le diazépane, N-méthyl homopipérazine, la N β-hydroxyéthylhomopipérazine et leurs sels d'addition.

Les hétérocycles pyrrolidine, 3-hydroxypyrrolidine, 3-aminopyrrolidine, 3-(méthylsulfonylamino)-pyrrolidine, proline, 3-hydroxyproline sont particulièrement préférés.

Selon un mode de réalisation particulier, R représente un radical alkyle, amino, hydroxyalkyle, hydroxy, R₄ est l'hydrogène, m est 0 ou 1, et n est compris entre 0 et 2.

A titre d'exemple, les composés de formule (1) peuvent être :
N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-hydroxyméthyl-3-hydroxypyrrolidine
N-(3,5-diaminopyridin-2-yl)-2-carboxamidopyrrolidine
N-(3,5-diaminopyridin-2-yl)-2-diméthylcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine
N-(3,5-diaminopyridin-2-yl)-2,5-diméthylpipéridine
N-(3,5-diaminopyridin-2-yl)-2-hydroxyméthylpipéridine
N-(3,5-diaminopyridin-2-yl)-2-carboxypipéridine
N-(3,5-diaminopyridin-2-yl)-3-hydroxypipéridine
N-(3,5-diaminopyridin-2-yl)-2-carboxamidopipéridine
N-(3,5-diaminopyridin-2-yl)-2-diméthylcarboxamidopipéridine
N-(3,5-diaminopyridin-2-yl)-4-hydroxypipéridine
N-(3,5-diaminopyridin-2-yl)homopipéridine
N-(3,5-diaminopyridin-2-yl)-2-carboxyhomopipéridine
N-(3,5-diaminopyridin-2-yl)-4-méthylpipérazine
N-(3,5-diaminopyridin-2-yl)-homopipérazine
N-(3,5-diaminopyridin-2-yl)-N'-méthylhomopipérazine et leurs sels d'addition.

N-(3,5-diaminopyridin-2-yl)-6-méthoxy-pyrrolidine
N-(3,5-diaminopyridin-2-yl)-6-méthoxy-2,5-diméthylpyrrolidine
N-(3,5-diaminopyridin-2-yl)-6-méthoxy-2-hydroxyméthylpyrrolidine
N-(3,5-diaminopyridin-2-yl)-6-méthoxy-proline
N-(3,5-diaminopyridin-2-yl)-6-méthoxy-3-hydroxypyrrolidine
N-(3,5-diaminopyridin-2-yl)-6-méthoxy-2-hydroxyméthyl-3-hydroxypyrrolidine
N-(3,5-diaminopyridin-2-yl)-6-méthoxy-2-carboxamidopyrrolidine
N-(3,5-diaminopyridin-2-yl)-6-méthoxy-2-diméthylcarboxamidopyrrolidine
N-(3,5-diaminopyridin-2-yl)-6-méthoxy-3,4-dihydroxypyrrolidine
N-(3,5-diaminopyridin-2-yl)-6-méthoxy-3-aminopyrrolidine
N-(3,5-diaminopyridin-2-yl)-6-méthoxy-3-diméthylaminopyrrolidine
N-(3,5-diaminopyridin-2-yl)-6-méthoxy-pipéridine
N-(3,5-diaminopyridin-2-yl)-6-méthoxy-2,5-diméthylpipéridine
N-(3,5-diaminopyridin-2-yl)-6-méthoxy-2-hydroxyméthylpipéridine
N-(3,5-diaminopyridin-2-yl)-6-méthoxy-2-carboxypipéridine
N-(3,5-diaminopyridin-2-yl)-6-méthoxy-3-hydroxypipéridine
N-(3,5-diaminopyridin-2-yl)-6-méthoxy-2-carboxamidopipéridine
N-(3,5-diaminopyridin-2-yl)-6-méthoxy-2-diméthylcarboxamidopipéridine
N-(3,5-diaminopyridin-2-yl)-6-méthoxy-4-hydroxypipéridine
N-(3,5-diaminopyridin-2-yl)-6-méthoxy-homopipéridine
N-(3,5-diaminopyridin-2-yl)-6-méthoxy-2-carboxyhomopipéridine
N-(3,5-diaminopyridin-2-yl)-6-méthoxy-homopipérazine
N-(3,5-diaminopyridin-2-yl)-6-méthoxy-N'-méthylhomopipérazine et leurs sels d'addition.

Les composés de formule (1) plus particulièrement préférés sont choisis parmi :
N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-carboxamidopyrrolidine
N-(3,5-diaminopyridin-2-yl)-2-diméthyfcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine
N-(3,5-diaminopyridin-2-yl)-4-méthylpipérazine
ainsi que leurs sels d'addition.

A titre de base d'oxydation hétérocyclique, on peut citer les pyridines, les pyrimidines, les pyrazoles, les pyrazolopyrimidines condensées, les pyrazolotriazoles, les pyrazolotetrazole, les pyrazolopyridazines, les pyrazolothiazoles, les pyrazoloimidazoles, les pyrazolopyrazoles, les pyrazoloimidazoles, les aminopyrazolones, les aminopyrolidine, les aminopyrazolines, les monoaminoquinoleines, les aminoindazoles, les diamino-uraciles, les hydrazones, les aminoindolénines, les diamino ou triaminoquinoleines, les dérivés de julolidine ou de lilolidine, les pyrazolobenzimidazoles.

A titre de diamino-uraciles , on peut citer les composés 5,6-diamino - uracile, 5,6-diamino-2-thio -uracile, 5,6-diamino-3-méthyl -uracile, 5-amino-3-méthyl-6-méthylamino -uracile, 5-amino-3-méthyl-6-bétahydroxyéthylamino -uracile, 5-amino-3-méthyl-6-benzylamino -uracile, 5-amino-3-méthyl-6-phénylamino -uracile, 5,6-diamino-1-phényl-uracile, 5,6-diamino-1 ,3-diméthyl -uracile, 5-amino-1,3-diméthyl-6-méthylamino -uracile, 5-amino-1 ,3-diméthyl-6-bétahydroxyméthylamino -uracile, 5-amino-1 ,3-diméthyl-6-benzyllamino -uracile, 5-amino-1,3-diméthyt-6-phényllamino - uracile, 5-amino-1 ,3-diméthyl-6-diméthylamino -uracile. De tels composés sont décrits dans le document DE 2533629.

A titre d'aminoindolénines, on peut citer les composés 2-méthyl-5-aminoindolénine, 1-bétahydroxyéthyl-2-méthyl-5-aminoindolénine. De tels composés sont décrits dans le document FR 1602547.

A titre de mono ou diaminotétrahydroquinoléines, on peut citer les composés 5-amino-1 ,2,3,4-tétrahydroquinoléine, 5-amino-7-chloro-8-pipéridino-1,2,3,4-tétrahydroquinoléine, 5-amino-7-chloro-8-morpholino-1,2,3,4-tétrahydroquinoléine, 5,7-diamino-6-méthyl-8-hydroxy-1,2,3,4-tétrahydroquinoléine, 5-amino-8-méthoxy-1,2,3,4-tétrahydroquinoléine, 5-amino-7-chloro-8-diméthylamino-1,2,3,4-tétrahydroquinoléine. De tels composés sont décrits dans le document DE 2441895.

A titre de diaminoquinoléines, on peut citer les composés 5,7-diamino-6-méthyl-8-hydroxyquinoléine et 5,7-diamino-2-méthyl-8-hydroxyquinoléine. De tels composés sont décrits dans le document DE 2441598.

A titre de triaminoquinoléine, on peut citer 5,7-diamino-8-méthylaminoquinoléine, 5,7-diamino-8-diméthylaminoquinoléine, 5,7-diamino-8-morpholinoquinoléine, 5,7-diamino-8-bétahydroxyéthylaminoquinoléine, 5,7,8-triaminoquinoléine. De tels composés sont décrits dans le document DE 2441599.

A titre d'aminopyrazolones, on peut citer l'acide 1-phényl-4-aminopyrazolone-3-carbonique, l'amide de l'acide 1-phényl-4-aminopyrazolone-3-carbonique, l'ester éthylique de l'acide 1-phényl-4-aminopyrazolone-3-carbonique, la 1-phényl-3-méthyl-4-aminopyrazolone. De tels composés sont décrits dans le document FR 1488169. On peut aussi citer la 3-amino-pyrazolone, 1-phényl-3-amino-pyrazolone, 1-phényl-3-hydroxy-pyrazolone. De tels composés sont décrits dans le document FR 1564999.

A titre de pyrazolothiazoles, on peut citer les composés 3-amino-2-méthyl-pyrazolo[3,2-b]thiazole, 3-amino-pyrazolo[3,2-b]thiazole, 3-amino-2,5-diméthyl-6-phénylpyrazolo[3,2-b]thiazole. De tels composés sont décrits dans le document US 5 427 200.

A titre de pyrazolotriazoles, on peut citer les composés 3-amino-4-méthyl-6-méthylthio-2-phényl-pyrazolo[3,2-c]-s-triazole, 3-amino-2,4,6-triméthyl-pyrazolo[3,2-c]-s-triazole, 3-amino-4,6-diméthyl-pyrazolo[3,2-c]-s-triazole. De tels composés sont décrits dans le document US 5 457 200. On peut aussi citer les composés 7-amino-2,6-diméthyl-pyrazolo[1,5-b]-1,2,4-triazole, 7-amino-3,6-diméthyl-pyrazolo[3,2-c]-1,2,4-triazole, 7-amino-3-méthyl-pyrazolo[3,2-c]-1,2,4-triazole, 7-amino-3-méthyl-6-carboxypyrazolo[3,2-c]-1,2,4-triazole, 7-amino-2-méthyl-pyrazolo[1,5-b]-1,2,4-triazole 7-amino-2-phényl-pyrazolo(1,5-b]-1,2,4-triazole, 7-amino-2-méthyl-6-carboxy-pyrazolo[1,5-b]-1,2,4-triazole. Ces composés sont décrits dans le EP 923929.

A titre de pyrazoloimidazoles, on peut citer les composés 3-amino-4-benzyl-pyrazolo[1,5-a]imidazole, 3-amino-2,4-diméthyl-pyrazolo[1,5-a]imidazole, 3-amino-4-méthyl-pyrazolo[1,5-a]imidazole. De tels composés sont décrits dans le document US 5457200. On peut aussi citer les composés 7-amino-6-méthyl-pyrazolo[1,5-a]imidazole, 7-amino-pyrazolo[1,5-a]imidazole, 7-amino-2-méthyl-pyrazolo[1,5-a]imidazole, 7-amino-2-phényl-pyrazolo[1,5-a]imidazole décrits dans le EP 923929.

A titre de pyrazoloquinolines, on peut citer les composés 3-amino-2-phényl-pyrazolo[1,5-a]quinoline. De tels composés sont décrits dans le document US 5 457200.

A titre de pyrazolopyridazine, on peut citer la 3-amino-pyrazolo[1,5-b]pyridazine. De tels composés sont décrits dans le document US 5 457200.

A titre de dérivés de julolidine ou de lilolidine, on peut citer les composés 9-aminojulolidine, 9-amino-8-méthyljulolidine, 9-amino-8,10-diméthyljulolidine, 8-aminolilolidine. De tels composés sont décrits dans le document DE 2441597.

A titre d'hydrazone, on peut citer les composés N-méthyl-pyridone-4-hydrazone, N-méthyl-thiazolone-hydrazone, N-méthyl-thiazolone-2-hydrazone, N,N-diméthyl-benzimidazolone-hydrazone, N-méthyl-pyridone-2-hydrazone, N-méthyl-benzothiazolone-2-hydrazone, 1,2-diméthyl-indazolone-3-hydrazone, 1,2,6-triméthyl-pyridone-4-hydrazone, 1-méthyl-quinolone-2-hydrazone, 1,2,6-triméthyl-3-nitropyridone-4-hydrazone, 1,2,6-triméthyl-3-aminopyridone-4-hydrazone, N-méthyl-cyclohexènothiazolone-hydrazone, 1,2,5-triméthyl-pyrazolone-3-hydrazone, 1,2-diméthyl-indazolone-3-hydrazone, 1,2-diméthyl-5-chloro-indazolone-3-hydrazone, 1-méthyl-2-éthyl-5-nitro-indazolone-3-hydrazone, N-méthyl-quinolone-4-hydrazone, N-méthyl-benzothiazolone-2-omégabenzènesulfonyl-hydrazone. De tels composés sont décrits dans le document FR 1 602 547.

A titre d'aminopyrazolines, on peut citer les composés 1-(4'-aminophényl)-3-aminopyrazoline, 1-(4'-hydroxyphényl)-3-aminopyrazoline. De tels composés sont décrits dans le document FR 2018056.

A titre d'aminoindazoles, on peut citer 4,7-diamino-5-méthylindazole, 4,7-diamino-5,6-diméthylindazole, 6,7-diaminoindazole, 6-hydroxy-7-aminoindazole, 1-éthyl-6-hydroxy-7-aminoindazole, 6-aminoindazole, 5,6-diaminoindazole. De tels composés sont décrits dans le document FR 2315906, DE 1492166.

A titre de pyrazolobenzimidazoles, on peut citer les composés 7-amino-6-méthyl-pyrazolo[1,5-a]benzimidazole, 6,7-diamino-pyrazolo[1,5-a]benzimidazole, 6,7-diamino-2-méthyl-pyrazolo[1,5-a]benzimidazole, 6,7-diamino-2-phényl-pyrazolo[1,5-a]benzimidazole décrits par exemple dans le EP 923929.

A titre de pyrazolotétrazoles, on peut citer les composés 7-amino-6-méthyl-pyrazolo[1,5-e]tétrazole, 7-amino-6-phényl-pyrazolo[1,5-e]tétrazole, 7-amino-6-carboxy-pyrazolo[1 ,5-e]tétrazole décrits dans le EP 923929.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-(1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

De préférence, les bases d'oxydation hétérocycliques utiles pour la présente invention sont choisies parmi les pyridines, les pyrimidines, les pyrazoles et les pyrazolopyrimidines.

Le rapport molaire entre la base hétéroaromatique et le coupleur est de préférence compris entre 0,01 et 100. Selon un mode de réalisation préféré, le rapport est compris entre 0,1 et 10.

Une composition tinctoriale pour la teinture des fibres kératiniques peut en outre comprendre une ou plusieurs bases d'oxydation classiques dans le domaine de la coloration. A titre d'exemple, on peut citer les paraphénylènediamines, les bis-phénylalkylènediamines, les para-aminophénols, les ortho-aminophénols et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthy) paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-((i-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

La ou les bases d'oxydation présentes dans la composition de l'invention sont en général présentent chacune en quantité comprise entre 0,001 et 10 % en poids environ du poids total de la composition tinctoriale, de préférence entre 0,005 et 6%.

La composition selon l'invention peut contenir un ou plusieurs coupleurs conventionnellement utilisés pour la teinture de fibres kératiniques autres que le coupleur pyridinique utile dans la présente invention. Parmi ces coupleurs, on peut notamment citer les métaphénylènediamines, les méta-aminophénols, les métadiphénols, les coupleurs naphtaléniques, les coupleurs hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont chacun généralement présents en quantité comprise entre 0,001 et 10 % en poids environ du poids total de la composition tinctoriale, de préférence entre 0,005 et 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Selon un mode de réalisation encore préféré, le pH est compris entre 6 et 8. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 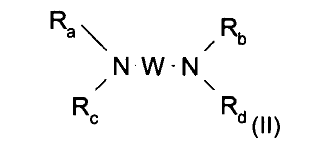 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé teinture des fibres kératiniques de la présente invention est un procédé dans lequel on applique sur les fibres la composition selon la présente invention telle que définie précédemment, en présence d'un agent oxydant pendant un temps suffisant pour développer la couleur désirée. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et préférentiellement entre 5 et 9. De façon encore préférée, le pH est compris entre 6 et 8. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale tel que défini précédemment, et l'application du mélange obtenu sur les fibres kératiniques pendant un temps suffisant pour développer la coloration désirée.

Ces coupleurs utiles dans la composition de l'invention peuvent être synthétisés selon le schéma de synthèse suivant : 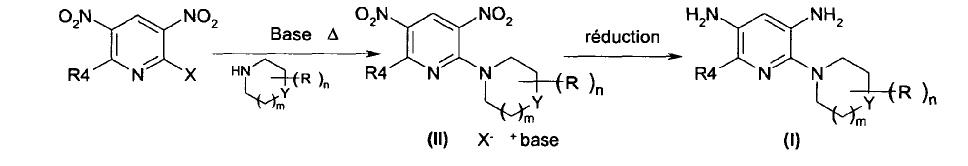 X représentant un atome d'halogène tel qu'un chlore ou brome, ou un radical alcoxy en C₁-C₂ et R4, R, n et m étant tels que définis précédemment.

Les composés de formule (II) peuvent être obtenus d'une façon générale en solubilisant sous agitation une 2-halogéno-3,5-dinitropyridine ou 2-alcoxy-3,5-dinitropyridine tel que la 2-chloro-3,5-dinitropyridine ou la 2-méthoxy-3,4-dinitropyridine dans un solvant protique ou aprotique de point d'ébullition compris entre 60°C et 180°C tel que par exemple le dioxane, le DMF, le THF, un alcool inférieur, l'eau et en présence d'une base organique ou inorganique pouvant former un sel avec l'ion libéré. L'amine cyclique est ensuite introduite au goutte à goutte. La température du milieu réactionnel est en général comprise entre 25°C et 100°C. Après disparition des réactifs, le milieu réactionnel est refroidi à température ambiante et versé sur un mélange de glace et d'eau. Le précipité ainsi formé est essoré sur fritté, lavé à l'eau puis il est séché sous vide jusqu'à poids constant.

Les composés de formule (I) peuvent ensuite être obtenus en réduisant les précurseurs nitrés de formule (II) soit par hydrogénation catalytique, soit par transfert d'hydrogène, soit par un métal tel que le zinc, l'étain ou le fer, soit par un hydrure tel que le borohydrure de sodium ou l'aluminohydrure de lithium. La réaction utilisée est de préférence l'hydrogénation catalytique hétérogène ou le transfert de phase par le cyclohexène. Le solvant est un solvant protique ou aprotique et de préférence un alcool de point d'ébullition compris entre 66°C et 160°C. Le catalyseur est classiquement le palladium sur charbon. La réaction d'hydrogénation est généralement réalisée à une température comprise entre 25°C et 80°C sous une pression d'hydrogène comprise entre 1 bar et 40 bars, de préférence entre 1 bar et 8 bars.

Les exemples qui suivent servent à illustrer l'invention sans toutefois présenter un caractère limitatif.

### EXEMPLES

### EXEMPLES DE TEINTURE

On a préparé les compositions tinctoriales suivantes (teneurs en mole):

| **Exemple** | **1** | **2** |
|---|---|---|
| 7-diméthylamino-3-amino-pyrazolopyrimidine | 2x10⁻³ mole | 1,5x10⁻² mole |
| 3,5-diamino-2-pyrrolidinopyridine | 2x10⁻³ mole | 1,5x10⁻² mole |
| Alcool benzylique | 2 g | 2 g |
| Polyéthylène glycol 8 OE | 3 g | 3 g |
| Ethanol | 18 g | 18 g |
| Alkyl (C8-C10) polyglucoside en solution aqueuse à 60% de matière active tamponné par du citrate d'ammonium vendu sous la dénomination ORAMIX CG110 par SEPPIC | 5 g en l'état | 5 g en l'état |
| Ammoniaque à 20% de NH₃ | 10 g | 10 g |
| Métabisulfite de sodium | 0,205 g | 0,205 g |
| Séquestrant | q.s. | q.s. |
| Eau déminéralisée | q.s.p. 100 g | q.s.p. 100 g |

Au moment de l'emploi, chaque composition est mélangée avec un poids égal d'eau oxygénée à 20 volumes (6% en poids).

Le mélange obtenu est appliqué sur des mèches de cheveux gris à 90 % de blancs, permanentés (BP) ou naturel (BN). Après 30 min de pose, les mèches sont rinçées, lavées avec un shampooing standard, rinçées à nouveau puis séchées.

A l'exemple 1, le reflet sur mèche est un gris neutre. A l'exemple 2, le reflet sur mèche est noir neutre.

## Revendications

1. Composition tinctoriale comprenant, dans un milieu de teinture cosmétique approprié, au moins une base hétérocyclique et au moins un coupleur pyridinique substitué en position 2,3,5 par un radical amino.

2. Composition selon la revendication 1 dans laquelle le coupleur pyridinique est un coupleur 2,3,5-triaminopyridinique de formule (I) : 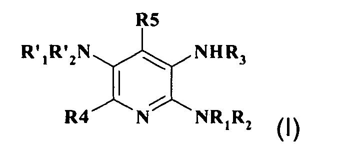 dans laquelle
• R₁, R₂, R'₁, R'₂, R₃ et R₅ représentent, indépendamment un atome d'hydrogène ; un radical alkyle linéaire ou ramifié de 1 à 6 atomes de carbone, éventuellement substitué par un ou plusieurs atomes d'halogène, un ou plusieurs radicaux hydroxy, amino, mono di ou tri alkyle(C1-C4)amino, aryle, alcoxyC₁-C₃ ou hétérocycle ; un radical aryl C₆-C₁₈, éventuellement substitué par un ou plusieurs radicaux amino, hydroxy, alcoxy(C₁-C₃) ou alkyl(C₁-C₆) ; un radical alcényle en C₂-C₆ ; un radical carboxyalkyl(C₁-C₆) ou un radical alkylthio(C₁-C₆), ou
• R₁ et R₂, et/ou R'₁, R'₂ ensemble, forment avec l'atome d'azote auquel ils sont rattachés un hétérocycle ayant de 3 à 18 chaînons, pouvant contenir un ou plusieurs hétéroatomes supplémentaires choisis parmi les atomes d'oxygène, d'azote ou de soufre, cet hétérocycle pouvant être substitué par un ou plusieurs radicaux alkyles en C₁-C₆, amino ou hydroxyalkyle(C₁-C₄), le cycle ne comportant pas de liaison peroxyde, ni de radicaux diazo ou nitroso, l'hétérocycle pouvant être substitué ;
• R₄ représente un atome d'hydrogène, un radical hydroxy, alcoxy, aryloxy, un atome d'halogène, un radical -OSO₂R", un radical -OCOR", un radical trifluoroalcoxy en C₁-C₃, avec R" représentant un atome d'hydrogène ou un radical alkyle.

3. Composition selon les revendications 1 ou 2 dans laquelle la base hétérocyclique est choisie parmi les bases les pyridines, les pyrimidines, les pyrazoles, les pyrazolopyrimidines condensées, les pyrazolotriazoles, les pyrazolotetrazole, les pyrazolopyridazines, les pyrazolothiazoles, les pyrazoloimidazoles, les pyrazolopyrazoles, les pyrazoloimidazoles, les aminopyrazolones, les aminopyrolidine, les aminopyrazolines, les monoaminoquinoleines, les aminoindazoles, les diamino-uraciles, les hydrazones, les aminoindolénines, les diamino ou triaminoquinoleines, les dérivés de julolidine ou de lilolidine, les pyrazolobenzimidazoles.

4. Composition selon l'une quelconque des revendications 1 à 3 dans laquelle la base d'oxydation est choisie parmi les pyridines, les pyrimidines, les pyrazoles et les pyrazolopyrimidines.

5. Composition selon l'une quelconque des revendications 1 à 4 dans laquelle le coupleur correspond à la formule : 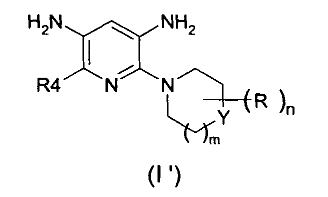 dans laquelle :
• R représente :
- un atome d'halogène ;
- un radical alkyle en C₁-C₄ éventuellement substitué par un ou plusieurs radicaux choisis parmi les radicaux hydroxy, carboxy, alcoxycarbonyle en C₁-C₄, carboxamido, alkyl(C₁-C₄)sulfonyle, un radical alcoxy en C₁-C₄, un radical alkyl(C₁-C₄)sulfonamido, un radical NR₆R₇;
- un radical carboxy ;
- un radical alcoxycarbonyle en C₁-C₄ ;
- un radical carboxamido ;
- un radical alkyl en C₁-C₄ carboxamido ;
- un radical sulfinique ;
- un radical alkyl(C₁-C₄)sulfonyle ;
- un radical alkyl(C₁-C₄)sulfonamido ;
- un radical hydroxy ;
- un radical alcoxy en C₁-C₄ ;
- un radical hydroxyalcoxy en C₂-C₄ ;
- un radical amino ou mono- ou di- amino alcoxy;
- un radical thioéther en C₁-C₄ ;
- un radical alkyl(C₁-C₄)sulfoxyde ;
- un radical sulfonique ;
- un radical NR₈R₉ ;
• R₆, R₇, R₈ et R₉ représentent, identiques ou différents, un atome d'hydrogène ; un radical alkyl(C₁-C₄)sulfonyle ; un radical alkyle(C₁-C₄)carbonyle dans lequel le radical alkyle peut être substitué par un ou plusieurs hydroxy ; un radical arylcarbonyle, le radical aryle pouvant être substitué par un radical choisi parmi hydroxy, alcoxy en C₁-C₄, amino ou (di)alkyl(C₁-C₄)amino ; un radical carboxamido ; un radical alkyle en C₁-C₄ éventuellement substitué par un ou plusieurs radicaux choisis parmi les radicaux hydroxy, alcoxy en C₁-C₄, alkyl(C₁-C₄)sulfonyle, alkyl(C₁-C₄)sulfonamido, carboxy, carboxamido, alkyl(C₁-C₄)sulfoxyde, amino ,(di)(C₁-C₄)alkylamino ou (poly)hydroxyalkylamino en C₂-C₄ ;
• R₄ représente un atome d'hydrogène ; un radical alcoxy en C₁-C₄ éventuellement substitué par un ou plusieurs radicaux hydroxy, alcoxy en C₁-C₂,
• n est un nombre entier compris entre 0 et 7, inclus,
• m est 0, 1 ou 2,
• Y représente un atome d'oxygène, un radical C(R₁₁)₂ ou un radical NR₁₀ où R₁₀ a la même signification que R₆ et R₁₁ identiques ou différents , représentent l'hydrogène ou ont la même signification que R.

6. Composition selon la revendication 5 dans laquelle R représente un radical alcoxy en C1-C4 éventuellement substitué par un ou plusieurs radicaux hydroxy, alcoxy en C1-C2, amino ou (di)alkylamino ; un radical hydroxy ; un radical amino ; un radical (di)alkylamino ; un radical alkyle en C1-C2 éventuellement substitué par un hydroxy, amino.

7. Composition selon la revendication 5 ou 6 dans laquelle R₄ représente un atome d'hydrogène ou un radical alcoxy.

8. Composition selon l'une quelconque des revendications 5 à 7 dans laquelle n représente 0 ou 1.

9. Composition selon l'une quelconque des revendications 5 à 8 dans laquelle R₆, R₇, R₈, R₉ et R₁₀ représentent, identiques ou différents, un atome d'hydrogène, un radical carboxamido, un radical alkyle en C₁-C₄ éventuellement substitué par un ou plusieurs radicaux choisis parmi les radicaux hydroxy, alcoxy en C₁-C₄, carboxamido, amino, (di)(C₁-C₄)alkylamino, (poly)hydroxyalkylamino en C₂-C₄.

10. Composition selon la revendication 9 dans laquelle R₆, R₇, R₈, R₉ et R₁₀ représentent, identiques ou différents, un atome d'hydrogène, un radical méthyle, un éthyle, 2-carboxyéthyte, 2-hydroxyéthyle, 3-hydroxypropyle, 2,3-dihydroxypropyle, 2-hydroxy-3-aminopropyle, 3-hydroxy-2-aminopropyle,

11. Composition selon la revendication 10 dans laquelle R₆, R₇, R₈, R₉ et R₁₀ représentent, identiques ou différents un atome d'hydrogène, un radical méthyle, un radical 2-hydroxyéthyle, un radical 2,3-dihydroxypropyle.

12. Composition selon l'une quelconque des revendications 5 à 11 dans laquelle l'azote en position 2 du cycle, Y et m forment un radical hétérocyclique choisi parmi les pyrrolidines, les pipéridines, les homopipéridines, les pipérazines, les homopipérazines, les diazépanes.

13. Composition selon la revendication 12 dans laquelle l'hétérocycle est choisi parmi la pyrrolidine, la 2,5-diméthylpyrrolidine, la 2-méthylpyrrolidine la proline, la 3-hydroxyproline, la 4-hydroxyproline, la 2,4-dicarboxypyrrolidine, la 2-hydroxyméthylpyrrolidine, la 3-hydroxy-2-hydroxyméthylpyrrolidine, la 2,5-di(hydroxyméthyl)pyrrolidine, la 2-carboxamidopyrrolidine, la 3-hydroxy-2-carboxamidopyrrolidine, la 2-(diméthylcarboxamido)pyrrolidine, la 2-(diméthylcarboxamido)-3-hydroxy-pyrrolidine, la 3,4-dihydroxy-2-hydroxyméthyl pyrrolidine, la 3-hydroxypyrrolidine, la 3,4-dihydroxypyrrolidine, la 3-aminopyrrolidine, la 3-méthylaminopyrrolidine, la 3-diméthylaminopyrrolidine, la 4-amino-3-hydroxy pyrrolidine, la 4-méthylamino-3-hydroxypyrrolidine, la 3-hydroxy-4-(2-hydroxyéthyl)amino-pyrrolidine, la pipéridine, la 2,6-diméthylpipéridine, la 2-carboxypipéridine, la 2-carboxamidopipéridine, la 2-(diméthylcarboxamido)pipéridine, la 2-hydroxyméthylpipéridine, la 3-hydroxy-2-hydroxyméthylpipéridine, 3-hydroxypipéridine, la 4-hydroxypipéridine, la 3-hydroxyméthylpipéridine, l'homopipéridine, la 2-carboxyhomopipéridine, la 2-carboxamidohomopipéridine, la pipérazine, la 4-méthylpipérazine le diazépane, N-méthyl homopipérazine, la N β-hydroxyéthylhomopipérazine et leurs sels d'addition.

14. Composition selon la revendication 13 dans laquelle l'hétérocycle est choisi parmi la pyrrolidine, la 2-méthylpyrrolidine, la 3-hydroxypyrrolidine, la 3-aminopyrrolidine, la 3-(méthylsulfonylamino)-pyrrolidine, la proline, la 3-hydroxyproline, la pipéridine, l'hydroxypipéridine, l'homopipéridine, la 4-méthylpipérazine, le diazépane, N-méthyl homopipérazine, la N β-hydroxyéthylhomopipérazine et leurs sels d'addition.

15. Composition selon la revendication 14 dans laquelle l'hétérocycle est choisi parmi la pyrrolidine, la 3-hydroxypyrrolidine, la 3-aminopyrrolidine, la 3-(méthylsulfonylamino)-pyrrolidine, la proline, la 3-hydroxyproline .

16. Composition selon la revendication 5 dans laquelle R représente un radical alkyle, amino, hydroxyalkyle, hydroxy, R₄ est l'hydrogène, m est 0 ou 1, et n est compris entre 0 et 2, inclus.

17. Composition selon l'une quelconque des revendications 5 à 16 dans laquelle les composés de formule (1) sont choisis parmi : N-(3,5-diaminopyridin-2-yl)pyrrolidine
N-(3,5-diaminopyridin-2-yl)-2-méthylpyrrolidine
N-(3,5-diaminopyridin-2-yl)-2,5-diméthylpyrrolidine
N-(3,5-diaminopyridin-2-yl)-2-hydroxyméthylpyrrolidine
N-(3,5-diaminopyridin-2-yl)proline
N-(3,5-diaminopyridin-2-yl)-3-hydroxypyrrolidine
N-(3,5-diaminopyridin-2-yl)-2-carboxamidopyrrolidine
N-(3,5-diaminopyridin-2-yl)-2-diméthylcarboxamidopyrrolidine
N-(3,5-diaminopyridin-2-yl)-3,4-dihydroxypyrrolidine
N-(3,5-diaminopyridin-2-yl)-3-aminopyrrolidine
N-(3,5-diaminopyridin-2-yl)-3-diméthylaminopyrrolidine
N-(3,5-diaminopyridin-2-yl)pipéridine .
N-(3,5-diaminopyridin-2-yl)-4-méthylpipérazine et leurs sels d'addition.

18. Composition selon l'une quelconque des revendications précédentes comprenant une base d'oxydation additionnelle choisie parmi les paraphénylènediamines, les bis-phénylalkylènediamines, les para-aminophénols, les ortho-aminophénols, et leurs sels d'addition.

19. Composition selon l'une quelconque des revendications précédentes comprenant un coupleur choisi parmi les métaphénylènediamines, les méta-aminophénols, les métadiphénols, les coupleurs naphtaléniques, les coupleurs hétérocycliques autres que les coupleurs de formule (1) et leurs sels d'addition.

20. Composition selon l'une quelconque des revendications précédentes dans laquelle la quantité de chacune des bases d'oxydation est comprise entre 0,001 à 10 % en poids environ du poids total de la composition tinctoriale.

21. Composition selon la revendication 17 dans laquelle la quantité de chacun des coupleurs est comprise entre 0,001 et 10 % en poids environ du poids total de la composition tinctoriale.

22. Procédé de teinture d'oxydation des fibres kératiniques, **caractérisé en ce qu'**on applique sur les fibres une composition tinctoriale telle que définie dans l'une quelconque des revendications 1 à 21 en présence d'un agent oxydant pendant un temps suffisant pour développer la couleur désirée.

23. Procédé selon la revendication 22 dans lequel l'agent oxydant est choisi parmi le peroxyde d'hydrogène, le peroxyde d'urée, les bromates de métaux alcalins, les persels, les peracides et les enzymes oxydases.

24. Dispositif à plusieurs compartiments dans lequel un premier compartiment contient une composition tinctoriale telle que définie à l'une quelconque des revendications 1 à 21 et un deuxième compartiment contient un agent oxydant.

25. Utilisation de la composition définie aux revendications 1 à 21 pour la teinture de fibres kératiniques.

26. Utilisation selon la revendication 25 pour la teinture des fibres kératiniques dans des nuances grise à noires.
